(19) Europäisches Patentamt
European Patent Office
Office européen des brevets

(11) **EP 2 851 119 A1**

(12) **EUROPEAN PATENT APPLICATION**
published in accordance with Art. 153(4) EPC

(43) Date of publication:
**25.03.2015 Bulletin 2015/13**

(21) Application number: **13790491.8**

(22) Date of filing: **16.05.2013**

(51) Int Cl.:
*B01J 27/199* (2006.01)   *B01J 37/04* (2006.01)
*B01J 37/08* (2006.01)   *C07C 51/235* (2006.01)
*C07C 57/055* (2006.01)   *C07B 61/00* (2006.01)

(86) International application number:
**PCT/JP2013/063669**

(87) International publication number:
**WO 2013/172414 (21.11.2013 Gazette 2013/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.05.2012 JP 2012114893**

(71) Applicant: **Nippon Kayaku Kabushiki Kaisha Tokyo 100-0005 (JP)**

(72) Inventors:
• **SAKAI, Hideomi**
  **Sanyoonoda-shi**
  **Yamaguchi 757-8686 (JP)**
• **NISHIMURA , Eiji**
  **Sanyoonoda-shi**
  **Yamaguchi 757-8686 (JP)**
• **EJIRI, Tomoyuki**
  **Sanyoonoda-shi**
  **Yamaguchi 757-8686 (JP)**

(74) Representative: **Gille Hrabal**
  **Brucknerstrasse 20**
  **40593 Düsseldorf (DE)**

(54) **CATALYST FOR USE IN PRODUCTION OF METHACRYLIC ACID, METHOD FOR PRODUCING SAID CATALYST, AND METHOD FOR PRODUCING METHACRYLIC ACID USING SAID CATALYST**

(57)   An object of the present invention is to provide a process for producing a catalyst for gas-phase contact oxidation of methacrolein, isobutyraldehyde or isobutyric acid to produce methacrylic acid in a high yield and a high selectivity. A catalyst wherein an alkali metal element, particularly cesium among alkali metal elements, is added by a specific method in a partially neutralized salt of a hetero polyacid which contains Mo, V, P, an alkali metal element and $NH_4$ as essential active ingredients, the catalyst being characterized by having extremely high catalytic performance.

EP 2 851 119 A1

## Description

Technical Field

The present invention relates to a catalyst for gas-phase contact oxidation of methacrolein, isobutyraldehyde or isobutyric acid to produce methacrylic acid using a catalyst having high activity and high selectivity, a process for producing the same, and a process for producing methacrylic acid using the catalyst.

Background Art

As catalysts to be used for gas-phase contact oxidation of methacrolein, isobutyraldehyde or isobutyric acid to produce methacrylic acid, a large number of catalysts have been proposed. These catalysts contain molybdenum and phosphorus as main ingredients and have a structure of a hetero polyacid and/or a salt thereof.

For example, as a process for preparing a catalyst for acrolein or methacrolein synthesis, Patent Document 1 discloses a preparation process wherein two or more kinds of solutions or dispersions containing catalyst ingredients are mixed for a short period of time as far as possible, thereafter the resulting mixture is immediately spray-dried without aging, and the resulting dried one is calcinated.

In Patent Document 2, it is described that upon preparing phosphorus, molybdenum, vanadium and arsenic-based catalysts, a catalyst having higher reaction activity, selectivity, catalyst strength and a long catalyst life in combination can be provided by previously preparing a slurry containing as a ingredient a Dawson-type hetero polyacid salt as a catalyst precursor, and subjecting it to concentration and drying and calcination to form a catalyst of a Keggin-type hetero polyacid.

As a preparation method of molybdenum, bismuth, iron-based complex oxide catalyst, Patent Document 3 describes a preparation method using a solution or dispersion containing a molybdenum compound as a first solution or dispersion and a solution or dispersion containing a bismuth compound as a second solution or dispersion and discloses large dependency on a mixing method and a subsequent heating and aging method, especially a stirring method of a liquid in each process.

These known technologies have attempted to achieve high activation of resulting catalysts through variously devising addition steps of the catalyst ingredients but the addition steps of the catalyst ingredients are complicated, so that establishment of a simple production process has been required. Furthermore, in the catalysts obtained by the processes, since the reaction activity is low, the selectivity to the objective substance is also low and the life is short, an improvement in performance of the catalysts has been desired although a part of the proposed catalysts have been industrially used.

In the preparation of a catalyst wherein a solution or slurry containing at least molybdenum, vanadium and phosphorus is mixed with a solution or slurry containing an ammonium radical, Patent Document 4 discloses a process for preparing a catalyst by discharging any one of the solution or slurry onto a continuous region of the liquid surface of another solution or slurry with stirring at a stirring power of 0.01 to 3.5 [$kW/m^3$], the continuous region occupying from 0.01 to 10% of the whole area of the liquid surface of the other solution or slurry, and drying a product.

However, even the above preparation process is not sufficient in view of satisfying both of the reaction yield (activity and selectivity) and the catalyst life and thus further improvement has been desired.

Background Art Document

Patent Document

    Patent Document 1: Japanese Patent No. 2847150
    Patent Document 2: Japanese Patent No. 3391532
    Patent Document 3: Japanese Patent No. 3288197
    Patent Document 4: WO05/039760

Summary of Invention

Problem that Invention is to Solve

An object of the present invention is to provide a process for producing a catalyst for gas-phase contact oxidation of methacrolein, isobutyraldehyde or isobutyric acid to produce methacrylic acid in a high yield and a high selectivity, a catalyst produced by the production process, and a process for producing methacrylic acid using the catalyst.

Means for Solving Problem

With regard to the catalyst of the invention, it has been found that a catalyst in which a Cs raw material is added by a specific method in a partially neutralized salt of a hetero polyacid containing Mo, V, P, Cs and $NH_4$ as essential active ingredients has an extremely high catalyst performance and thus the present invention has been accomplished.
Namely, the invention relates to:

(1) A process for producing a catalyst for methacrylic acid production, comprising the following steps:

Step a): a step of preparing a slurry containing at least molybdenum, phosphorus and vanadium;
Step b): a step of cooling the slurry obtained in Step a) and subsequently adding an alkali metal compound with stirring of the slurry to prepare a slurry of a partially neutralized salt of a hetero polyacid;
Step c): a step of adding an ammonium compound to the slurry obtained in Step b);
Step d): a step of drying the slurry, to which the ammonium compound has been added, obtained in Step c) to obtain a dry powder having a composition shown by the following (Formula 1);
Step e): a step of shaping the dry powder obtained in Step d); and
Step f): a step of calcinating the shaped product obtained in Step e):

$$Mo_{10}V_aP_b(NH_4)_cX_dY_eO_f \qquad \text{(Formula 1)}$$

wherein Mo represents molybdenum; V represents vanadium; P represents phosphorus; ($NH_4$) represents an ammonium group; X represents at least one alkali metal element selected from the group consisting of K, Rb and Cs; Y represents at least one element selected from the group consisting of Sb, As, Cu, Ag, Mg, Zn, Al, B, Ge, Sn, Pb, Ti, Zr, Cr, Re, Bi, W, Fe, Co, Ni, Ce and Th; O represents oxygen; a to e represents atomic ratios of respective elements, a: $0.1 \leq a \leq 6.0$, b: $0.5 \leq b \leq 6.0$, c: $0.1 \leq c \leq 10.0$, d: $0.1 \leq d \leq 3.0$, e: $0 \leq e \leq 3$, and f is a value other than 0, which is determined depending on oxidation states and atomic ratios of individual elements.

(2) The process for producing a catalyst for methacrylic acid production as described in (1) above,
wherein in the above Step b), the alkali metal compound is added with stirring at a power requirement for stirring per unit volume Pv [$kW/m^3$] of 0.01 to 4.00 [$kW/m^3$].
(3) The process for producing a catalyst for methacrylic acid production as described in (1) or (2) above,
wherein temperature of the slurry containing at least molybdenum, phosphorus and vanadium is controlled to 0 to 35°C at the time of adding the alkali metal compound in the above Step b).
(4) The process for producing a catalyst for methacrylic acid production as described in any one of (1) to (3) above,
wherein the above Step e) is a step of coating an inert support with the dry powder using a binder to form a coated catalyst.
(5) The process for producing a catalyst for methacrylic acid production as described in (4) above,
wherein the binder is at least one kind of liquid selected from the group consisting of water and organic compounds having a boiling point of 150°C or lower under 1 atm.
(6) The process for producing a catalyst for methacrylic acid production as described in any one of (1) to (5) above,
wherein calcination temperature in Step f) is from 100 to 450°C.
(7) A catalyst for methacrylic acid production, which is obtained by the process for producing a catalyst for methacrylic acid production as described in any one of (1) to (6) above.
(8) A process for producing methacrylic acid, the process comprising:
conducting gas-phase contact oxidation of at least one selected from the group consisting of methacrolein, isobutyraldehyde and isobutyric acid using the catalyst as described in (7) above.

Effects of Invention

According to the invention, it is possible to provide a highly active and highly selective catalyst containing molybdenum, phosphorus, vanadium, a specific alkali metal element and ammonia as essential ingredients.

Mode for Carrying Out Invention

The catalyst for methacrylic acid production capable of being produced by the production process of the invention is used at the production of methacrylic acid by gas-phase contact oxidation of methacrolein or the like with molecular oxygen and has the following composition represented by the following (Formula 1).

$$Mo_{10}V_aP_b(NH_4)_dC_dY_eO_f \qquad \text{(Formula 1)}$$

In the above (Formula 1), Mo represents molybdenum, V represents vanadium, P represents phosphorus, (NH$_4$) represents an ammonium group, X represents at least one element selected from the group consisting of K, Rb and Cs, Y represents at least one alkali metal element selected from the group consisting of Sb, As, Cu, Ag, Mg, Zn, Al, B, Ge, Sn, Pb, Ti, Zr, Cr, Re, Bi, W, Fe, Co, Ni, Ce and Th, a to e represents atomic ratios of respective elements, a: 0.1≤a≤6.0, preferably 0.3≤a≤2.0, b: 0.≤b≤6.0, preferably 0.7≤b≤2.0, c: 0.1≤c≤10.0, preferably 0.5≤C≤5.0, d: 0.1≤d≤3.0, preferably 0.4≤d≤1.5, e: 0≤e≤3, preferably 0.01≤e≤0.5, and f is a value other than 0, which is determined depending on the oxidation states and atomic ratios of individual elements.

In the above (Formula 1), the X ingredient is preferably Cs and the Y ingredient is preferably at least one element selected from the group consisting of Sb, As and Cu.

The following will describe preferable embodiments according to the above individual steps.

Step a) First, as the active ingredient-containing compound(s) for use in catalyst preparation, chlorides, sulfates, nitrates, oxides, acetates or the like of the active ingredient elements may be mentioned. When preferable compounds are more specifically exemplified, there may be mentioned nitrates such as potassium nitrate or cobalt nitrate; oxides such as molybdenum oxide, vanadium pentoxide, antimony trioxide, cerium oxide, zinc oxide, or germanium oxide; acids (or salts thereof) such as orthophosphoric acid, phosphoric acid, boric acid, aluminum phosphate, or 12 tungstophosphoric acid, and the like. These active ingredient-containing compounds may be used singly or two or more thereof may be used as a mixture. The slurry can be obtained by homogeneously mixing the active ingredient-containing compound(s) with water. The amount of water to be used in the slurry is not particularly limited so long as it is an amount with which the total amount of the compounds to be used can be completely dissolved or can be homogeneously mixed. The amount is appropriately determined in consideration of the drying method, drying conditions, and the like. Usually, based on 100 parts by mass of the total mass of the compounds for slurry preparation, about 200 to 2,000 parts by mass of water is used. The amount of water may be a large amount but when the amount is too large, there are such many demerits that an energy cost for the drying step increases, there arises a case where complete drying is impossible, and the like.

As a temperature at the preparation of the slurry, it is preferred to heat the mixture to a temperature at which compounds containing molybdenum, phosphorus, vanadium and, if necessary, other metal elements can be thoroughly dissolved.

The alkali metal compound to be added in Step b) is preferably cesium and, as a cesium-containing compound, cesium hydroxide or a cesium weak acid salt such as cesium acetate or cesium carbonate.

As an ammonium compound to be used in Step c), ammonium acetate or ammonium hydroxide is preferred.

In Steps b) and c), the temperature of the slurry containing at least molybdenum, phosphorus and vanadium is the range of usually 0 to 35°C, preferably about 0 to 30°C. In this case, the resulting catalyst tends to be highly active.

In the case of adding the Y ingredient, the step for the addition is not particularly limited and the ingredient may be appropriately added in any step from Step a) to Step e). As raw materials of the Y ingredient, chlorides, sulfates, nitrates, oxides, acetates or the like of the ingredient elements may be mentioned.

At the time of mixing the alkali metal compound in the above Step b), the slurry prepared in Step a) is stirred at a power requirement for stirring per unit volume Pv [kW/m$^3$] of preferably 0.01 to 4.00 [kW/m$^3$], more preferably 0.1 to 3.75 [kW/m$^3$], further preferably 0.5 to 3.50 [kW/m$^3$]. When the power requirement for stirring per unit volume Pv is less than 0.01 [kW/m$^3$], stirring ability extremely decreases and there is a case where a stable catalyst is not obtained. On the other hand, when the power requirement for stirring per unit volume Pv exceeds 4.00 [kW/m$^3$], not only the energy cost exceedingly increases but also the activity of the resulting catalyst becomes too high, so that there is a case where the yield of objective methacrylic acid decreases as a result. The reason is not clear but the inventors surmise that this is because the degree of dispersion of cesium becomes exceedingly high under high stirring power.

Here, the power requirement for stirring per unit volume Pv is a quotient obtained by dividing the power requirement for stirring P by the volume V in a mixing tank as shown in JP-A-2002-113303 and is calculated according to the following (Formula 2). The unit of the volume V is m$^3$ (cubic m) and the unit of the power requirement for stirring per unit volume Pv is kW/m$^3$.

$$\text{Power requirement for stirring per unit volume Pv} = \text{Power requirement for stirring/Volume V} \qquad \text{(Formula 2)}$$

In the invention, the shape of the stirring blade of a stirrer for use at the time of adding the essential active ingredients is not particularly limited and any stirring blade such as a propeller blade, a turbine blade, a paddle blade, a tilt-paddle blade, a screw blade, an anchor blade, a ribbon blade or a large lattice blade can be used in a single stage or the same or different blades can be used in two or more stages in a perpendicular direction. Moreover, a baffle (baffle plate) may be provided in the reaction tank according to needs.

The drying method in the above Step d) is not particularly limited so long as it is a method capable of completely drying the slurry. For example, drum drying, freeze drying, spray drying, evaporation to dryness and the like may be mentioned. Of these, in the invention, the spray drying capable of drying the slurry into a powder or granules for a short period of time is particularly preferred. The drying temperature in the spray drying varies depending on the concentration, liquid-transferring rate and the like of the slurry but the outlet temperature of a drier is generally from 70 to 150°C. On this occasion, drying is preferably performed so that the average particle size of the resulting slurry-dried matter becomes from 10 to 700 $\mu$m.

The shaping method in the above Step e) is not particularly limited but it is preferred to shape the dry powder into columnar articles, pellets, ring form ones, spherical ones or the like, in order to reduce pressure loss of a reactive gas in the oxidation reaction at the production of methacrylic acid. Particularly, since improvement in selectivity and removal of reaction heat can be expected, it is particularly preferred that an inactive support is coated with the slurry-dried matter to form a coated catalyst. In the coating step, a rolling granulation method to be described below is preferred. The method is a method where, for example, in an apparatus having a flat or uneven disk at the bottom of a fixed container, the support in the container is vigorously stirred through repetition of autorotation movement and orbital movement by rotating the disk at a high speed and the support is coated herein with a mixture for coating obtained by adding a binder and the slurry-dried powder and, if necessary, other additives such as a shaping aid and a strength enhancer.

As methods of adding the binder, methods of 1) mixing it into the mixture for coating beforehand, 2) adding it at the same time when the mixture for coating is added into the fixed container, 3) adding the binder after the mixture for coating is added into the fixed container, 4) adding the binder before the mixture for coating is added into the fixed container, 5) dividing each of the mixture for coating and the binder and adding all the amounts of them with appropriately combining 2) to 4), and the like may be arbitrarily adopted. Of these, in the method of 5), for example, it is preferred to perform the addition with regulating addition rates using an auto feeder or the like so that a prescribed amount of the mixture for coating is supported on the support without attachment of the mixture for coating to walls of the fixed container and aggregation of the mixture for coating itself

The binder is not particularly limited so long as it is at least one selected from the group consisting of water and organic compounds having a boiling point of 150°C or lower under 1 atm or less. Specific examples of the binders other than water include alcohols such as methanol, ethanol, propanols and butanols, preferably alcohols having 1 to 4 carbon atoms, ethers such as ethyl ether, butyl ether and dioxane, esters such as ethyl acetate and butyl acetate, ketones such as acetone and methyl ethyl ketone and aqueous solutions thereof and particularly, ethanol is preferred. In the case where ethanol is used as the binder, preferred is ethanol/water = 10/0 to 0/10 (mass ratio), preferably 9/1 to 1/9 (mass ratio) in which ethanol is mixed with water. The amount of these binders to be used is usually from 2 to 60 parts by mass, preferably from 10 to 50 parts by mass based on 100 parts by mass of the mixture for coating. When the catalyst active ingredient solid obtained in the step is shaped after calcination at about 250°C to 350°C, there is a case where the mechanical strength and catalyst performance are enhanced, so that the case is preferred.

Specific examples of the support in the above coating include spherical supports having a diameter of 1 to 15 mm, preferably 2.5 to 10 mm, made of silicon carbide, alumina, silica-alumina, mullite, alundum and the like. As these supports, those having a porosity of 10 to 70% are usually employed. Regarding the ratio of the support to the mixture for coating, there are used amounts so that mixture for coating/(mixture for coating + support) is usually 10 to 75% by mass, preferably 15 to 60% by mass. When the ratio of the mixture for coating is large, the reaction activity of the coated catalyst increases but the mechanical strength tends to decrease. On the other hand, when the ratio of the mixture for coating is small, the mechanical strength is large but the reaction activity tends to decrease. Incidentally, in the above, as the shaping aid to be used according to needs, silica gel, diatomaceous earth, alumina powder and the like may be mentioned. The amount of the shaping aid to be used is usually from 1 to 60 parts by mass based on 100 parts by mass of the dry powder. Moreover, it is useful for enhancing mechanical strength of the catalyst to use further inorganic fibers (e.g., ceramic fibers or whiskers) inactive to the catalyst ingredients and the reaction gas as a strength enhancer according to needs and particularly, glass fibers are preferred. The amount of the fibers to be used is usually from 1 to 30 parts by mass based on 100 parts by mass of the dry powder.

The coated catalyst obtained in Step f) and Step e) can be used in the gas-phase contact oxidation reaction as it is as a catalyst but there is a case where the catalyst activity is enhanced when it is calcined, so that the case is preferred. The calcination temperature in this case is usually from 100 to 450°C, preferably 250°C to 420°C, more preferably 250 to lower than 400°C, and further preferably from 300 to lower than 400°C. The calcination time is from 1 to 20 hours. Incidentally, the calcination is usually performed under an air atmosphere but may be performed under an atmosphere of an inert gas such as nitrogen or under an atmosphere of a reducing gas such as ethanol. After calcination under an inert gas atmosphere or a reducing gas atmosphere, the calcination may be further performed under an air atmosphere according to needs. The ratio of the active ingredients to the whole coated catalyst obtained as above is from 10 to 60% by mass.

The thus obtained catalyst (hereinafter referred to as catalyst of the invention) is used in the production of methacrylic acid by gas-phase contact oxidation of methacrolein, isobutyraldehyde or isobutyric acid. The following will describe the gas-phase contact oxidation reaction using methacrolein that is the most preferable raw material for the use of the catalyst of the invention. In the gas-phase contact oxidation reaction, molecular oxygen or a molecular oxygen-containing gas is used. The ratio of the molecular oxygen to be used to methacrolein is preferably the range of 0.5 to 20 and particularly preferably the range of 1 to 10 as a molar ratio. For the purpose of smooth proceeding of the reaction, it is preferred to add water into a raw material gas in the range of 1 to 20 as a molar ratio. The raw material gas may contain an inert gas inactive to the reaction, such as nitrogen, carbon dioxide or a saturated hydrocarbon, and the like according to needs, other than oxygen and, if necessary, water (usually contained as water vapor). Moreover, methacrolein may be supplied as a gas obtained in the oxidation of isobutylene, tertiary butanol and methyl tertiary butyl ether without further treatment. The reaction temperature in the gas-phase contact oxidation reaction is usually from 200 to 400°C and preferably from 260 to 360°C and the supplying amount of the raw material gas is usually from 100 to 6,000 $hr^{-1}$ and preferably from 300 to 3,000 $hr^{-1}$ as a space velocity (SV). Furthermore, the gas-phase contact oxidation reaction can be carried out under pressurization or under reduced pressure but generally, a pressure around atmospheric pressure is suitable.

Examples

The following will more specifically describe the invention with reference to Examples but the invention should not be construed as being limited to Examples.

In the following, the conversion, selectivity and yield are defined as follows.

$$\text{Conversion} = (\text{Number of moles of reacted methacrolein/Number of moles of supplied methacrolein}) \times 100$$

$$\text{Selectivity} = (\text{Number of moles of formed methacrylic acid/Number of moles of reacted methacrolein}) \times 100$$

$$\text{Yield} = (\text{Number of moles of formed methacrylic acid/Number of moles of supplied methacrolein}) \times 100$$

Example 1

1) Preparation of Catalyst

To 5,680 ml of pure water were added 800 g of molybdenum trioxide, 30.33 g of vanadium pentoxide, and 76.87 g of 85% by mass orthophosphoric acid, and the whole was stirred at 92°C for 3 hours to obtain a reddish brown transparent solution. Then, the solution was cooled to 0 to 20°C and 661.32 g of a 9.1 % by mass aqueous cesium hydroxide solution was gradually added thereto under stirring at a power requirement for stirring per unit volume Pv of 0.77 [kW/m³] and aging was performed at 15 to 20°C for 1 hour to obtain a yellow slurry. Subsequently, 196.86 g of a 50.0% by mass aqueous ammonium acetate solution was gradually added to the slurry and aging was further performed at 0 to 30°C for 1 hour. Then, further, 22.18 g of cupric acetate was added to the slurry and the whole was stirred and mixed at 0 to 30°C until complete dissolution. Subsequently, the slurry was spray-dried to obtain a catalyst active ingredient solid. Composition of the catalyst active ingredient solid determined from the charged amounts of the raw materials is as follows:

$$Mo_{10}V_{0.6}P_{1.1}Cs_{0.7}(NH_4)_{2.3}Cu_{0.3}.$$

Then, 120 g of the catalyst active ingredient solid and 6.5 g of a strength enhancer (glass fibers) were homogeneously mixed and 200 g of a spherical porous alumina support (particle size: 4.5 mm) was coat-shaped therewith using about 30 g of a 50% by mass aqueous ethanol solution as a binder. Subsequently, the resulting shaped article was calcinated at 380°C over a period of 5 hours under an air flow to obtain an objective coated catalyst. With regard to the active ingredient composition after calcination, it is considered that the ammonia ingredient is almost lost by calcination and becomes from about 0.01 to 1.0.

2) Catalytic Oxidation Reaction of Methacrolein

Into a stainless steel reaction tube having an inner diameter of 18.4 mm was packed 10.3 ml of the resulting coated catalyst, and an oxidation reaction of methacrolein was carried out with a raw material gas (composition (molar ratio): methacrolein:oxygen:water vapor:nitrogen = 1:2:4:18.6) under conditions of a space velocity (SV) of 1,200 hr$^{-1}$ and a reaction bath temperature of 310°C. The reaction was first continued at a reaction bath temperature of 310°C for 3 hours, then the reaction bath temperature was elevated to 350°C, and the reaction was continued for 15 hours (hereinafter, the treatment is referred to as high-temperature reaction treatment). Then, the reaction bath temperature was lowered to 310°C and measurement of reaction performance was conducted. Results obtained are shown in Table 1.

Example 2

A coated catalyst was prepared in the same manner as in Example 1 except that stirring was performed at a power requirement for stirring per unit volume Pv of 3.1 [kW/m$^3$]. Results obtained are shown in Table 1.

Comparative Example 1

A coated catalyst was prepared in the same manner as in Example 1 except that addition was performed while stirring at the addition of the aqueous cesium hydroxide solution was stopped in Example 1. Results obtained are shown in Table 1.

Example 3

A coated catalyst was prepared in the same manner as in Example 1 except that all the raw materials were scaled up by 230 times and stirring was performed at a power requirement for stirring per unit volume Pv of 1.8 [kW/m$^3$] in Example 1. Results obtained are shown in Table 1.

Comparative Example 2

A coated catalyst was prepared in the same manner as in Example 1 except that the preparation of the catalyst active ingredients was as follows.
To 5,680 ml of pure water were added 800 g of molybdenum trioxide, 30.33 g of vanadium pentoxide, and 76.87 g of 85% by mass orthophosphoric acid, and the whole was stirred at 92°C for 3 hours to obtain a reddish brown transparent solution. Then, the solution was cooled to 0 to 20°C and 196.86 g of a 50.0% by mass aqueous ammonium acetate solution was gradually added thereto under stirring at a power requirement for stirring per unit volume Pv of 2.1 [kW/m$^3$] and aging was performed at 15 to 20°C for 1 hour to obtain an orange slurry. Subsequently, 661.32 g of a 9.1% by mass aqueous cesium hydroxide solution was gradually added to the slurry and aging was further performed at 0 to 30°C for 1 hour. Then, further, 22.18 g of cupric acetate was added to the slurry and the whole was stirred and mixed at 0 to 30°C until complete dissolution.
Results obtained are shown in Table 1.
[Table 1]

Table 1

| | Power requirement for stirring kW/m$^3$ | Ratio of methacrolein % | Selectivity for methacrylic acid % | Yield of methacrylic acid % |
|---|---|---|---|---|
| Example 1 | 0.77 | 89.26 | 84.06 | 75.03 |
| Example 2 | 3.1 | 91.21 | 82.59 | 75.33 |
| Example 3 | 1.8 | 91.46 | 82.45 | 75.41 |

(continued)

| | Power requirement for stirring kW/m³ | Ratio of methacrolein % | Selectivity for methacrylic acid % | Yield of methacrylic acid % |
|---|---|---|---|---|
| Comparative Example 1 | 0 | 78.12 | 88.86 | 69.42 |
| Comparative Example 2 | 2.1 | 67.60 | 90.08 | 60.90 |

While the invention has been described in detail and with reference to specific embodiments thereof, it will be apparent to one skilled in the art that various changes and modifications can be made therein without departing from the spirit and scope thereof.

Incidentally, the present application is based on Japanese Patent Application No. 2012-114893 filed on May 18, 2012, and the contents are incorporated herein by reference. Also, all the references cited herein are incorporated as a whole.

Industrial Applicability

According to the present invention, it is possible to provide a catalyst for gas-phase contact oxidation of methacrolein, isobutyraldehyde or isobutyric acid to produce methacrylic acid in a high yield and a high selectivity.

**Claims**

1.   A process for producing a catalyst for methacrylic acid production, comprising the following steps:

Step a): a step of preparing a slurry containing at least molybdenum, phosphorus and vanadium;
Step b): a step of cooling the slurry obtained in Step a) and subsequently adding an alkali metal compound with stirring of the slurry to prepare a slurry of a partially neutralized salt of a hetero polyacid;
Step c): a step of adding an ammonium compound to the slurry obtained in Step b);
Step d): a step of drying the slurry, to which the ammonium compound has been added, obtained in Step c) to obtain a dry powder having a composition shown by the following (Formula 1);
Step e): a step of shaping the dry powder obtained in Step d); and
Step f): a step of calcinating the shaped product obtained in Step e):

$$Mo_{10}V_aP_b(NH_4)_cX_dY_eO_f \qquad \text{(Formula 1)}$$

wherein Mo represents molybdenum; V represents vanadium; P represents phosphorus; $(NH_4)$ represents an ammonium group; X represents at least one alkali metal element selected from the group consisting of K, Rb and Cs; Y represents at least one element selected from the group consisting of Sb, As, Cu, Ag, Mg, Zn, Al, B, Ge, Sn, Pb, Ti, Zr, Cr, Re, Bi, W, Fe, Co, Ni, Ce and Th; O represents oxygen; a to e represents atomic ratios of respective elements, a: $0.1 \leq a \leq 6.0$, b: $0.5 \leq b \leq 6.0$, c: $0.1 \leq c \leq 10.0$, d: $0.1 \leq d \leq 3.0$, e: $0 \leq e \leq 3$, and f is a value other than 0, which is determined depending on oxidation states and atomic ratios of individual elements.

2.   The process for producing a catalyst for methacrylic acid production according to claim 1, wherein in the Step b), the alkali metal compound is added with stirring at a power requirement for stirring per unit volume Pv [kW/m³] of 0.01 to 4.00 [kW/m³].

3.   The process for producing a catalyst for methacrylic acid production according to claim 1 or 2, wherein temperature of the slurry containing at least molybdenum, phosphorus and vanadium is controlled to 0 to 35°C at the time of adding the alkali metal compound in the above Step b).

4.   The process for producing a catalyst for methacrylic acid production according to any one of claims 1 to 3, wherein the above Step e) is a step of coating an inert support with the dry powder using a binder to form a coated catalyst.

**5.** The process for producing a catalyst for methacrylic acid production according to claim 4, wherein the binder is at least one kind of liquid selected from the group consisting of water and organic compounds having a boiling point of 150°C or lower under 1 atm.

**6.** The process for producing a catalyst for methacrylic acid production according to any one of claims 1 to 5, wherein calcination temperature in Step f) is from 100 to 450°C.

**7.** A catalyst for methacrylic acid production, which is obtained by the process for producing a catalyst for methacrylic acid production according to any one of claims 1 to 6.

**8.** A process for producing methacrylic acid, the process comprising:

conducting gas-phase contact oxidation of at least one selected from the group consisting of methacrolein, isobutyraldehyde and isobutyric acid using the catalyst according to claim 7.

## INTERNATIONAL SEARCH REPORT

| International application No. |
| --- |
| PCT/JP2013/063669 |

| A. CLASSIFICATION OF SUBJECT MATTER |
| --- |
| *B01J27/199*(2006.01)i, *B01J37/04*(2006.01)i, *B01J37/08*(2006.01)i, *C07C51/235*(2006.01)i, *C07C57/055*(2006.01)i, *C07B61/00*(2006.01)n |

According to International Patent Classification (IPC) or to both national classification and IPC

| B. FIELDS SEARCHED |
| --- |
| Minimum documentation searched (classification system followed by classification symbols) |
| B01J27/199, B01J37/04, B01J37/08, C07C51/235, C07C57/055, C07B61/00 |

| Documentation searched other than minimum documentation to the extent that such documents are included in the fields searched |
| --- |
| Jitsuyo Shinan Koho 1922-1996 Jitsuyo Shinan Toroku Koho 1996-2013 |
| Kokai Jitsuyo Shinan Koho 1971-2013 Toroku Jitsuyo Shinan Koho 1994-2013 |

| Electronic data base consulted during the international search (name of data base and, where practicable, search terms used) |
| --- |
| |

### C. DOCUMENTS CONSIDERED TO BE RELEVANT

| Category* | Citation of document, with indication, where appropriate, of the relevant passages | Relevant to claim No. |
| --- | --- | --- |
| X<br>Y | WO 2010/052909 A1 (Nippon Kayaku Co., Ltd.),<br>14 May 2010 (14.05.2010),<br>paragraphs [0032], [0033]<br>& CN 102203040 A & KR 10-2011-0092272 A | 1,3-8<br>2 |
| Y | WO 2005/039760 A1 (Mitsubishi Rayon Co., Ltd.),<br>06 May 2005 (06.05.2005),<br>claims 1, 2; paragraph [0035]<br>& US 2007/0032679 A1 & CN 1874842 A<br>& KR 10-2006-0076320 A | 2 |

☒ Further documents are listed in the continuation of Box C.  ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 26 July, 2013 (26.07.13) | 06 August, 2013 (06.08.13) |

| Name and mailing address of the ISA/<br>Japanese Patent Office | Authorized officer |
| --- | --- |
| Facsimile No. | Telephone No. |

Form PCT/ISA/210 (second sheet) (July 2009)

**INTERNATIONAL SEARCH REPORT**

International application No.

PCT/JP2013/063669

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-272151 A (Nippon Kayaku Co., Ltd.), 12 October 2006 (12.10.2006), paragraphs [0020], [0021] & US 2009/0234158 A1 & EP 1867387 A1 & EP 2204234 A1 & WO 2006/104155 A1 & KR 10-2007-0114362 A & CN 101175568 A & CN 101829589 A & KR 10-2012-0043152 A | 1-8 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2847150 B
- JP 3391532 B
- JP 3288197 B
- WO 05039760 A
- JP 2002113303 A
- JP 2012114893 A